# EUROPEAN PATENT APPLICATION

(11) **EP 4 831 203 A1**
(43) Date of publication of application: **28.10.2026**
(21) Application number: 24906597.0
(22) Date of filing: 25.12.2024
(51) Int. Cl.: C07K 16/28, C12N 15/13, A61K 39/395, A61P 35/00, A61P 37/02

(54) **ANTI-CD5 SINGLE DOMAIN ANTIBODY AND APPLICATION THEREOF**

(30) Priority: 20.12.2023 CN 202311763267
(71) Applicant: Shenzhen Pregene Biopharma Co., Ltd., Shenzhen, Guangdong 518000 (CN)
(72) Inventor: ZHANG, Jishuai, Shenzhen, Guangdong 518122 (CN); LI, Hongjian, Shenzhen, Guangdong 518122 (CN); SU, Hongchang, Shenzhen, Guangdong 518122 (CN); ZU, Yingling, Shenzhen, Guangdong 518122 (CN); LUO, Yanping, Shenzhen, Guangdong 518122 (CN)
(74) Representative: Winter, Brandl - Partnerschaft mbB
(86) International application number: PCT/CN2024/142307
(87) International publication number: WO 2025/131127

(57) **Abstract**

An anti-CD5 single domain antibody and an application thereof, which belong to the technical field of antibodies. Heavy chain complementarity determining regions of the provided anti-CD5 single domain antibody comprise CDR1, CDR2, and CDR3. The provided anti-CD5 single domain antibody can specifically bind to CD5, and can recognize an extracellular region D3 juxtamembrane domain of a CD5 molecule. Further provided is a humanized anti-CD5 single domain antibody, which reduces the immunogenicity of a molecule in the body, and the humanized anti-CD5 single domain antibody can specifically recognize a CD5 molecule and has relatively high affinity. The present invention provides broad prospects for the development of drugs targeting CD5.

## Description

The present invention claims the priority of the Chinese patent application filed with the Chinese Patent Office on December 20, 2023, with application number 202311763267.1 and title "Anti-CD5 single domain antibody and application thereof". Its entire content is incorporated by reference herein.

### TECHNICAL FIELD

This invention belongs to the technical field of antibody, specifically relating to anti-CD5 single domain antibody and application thereof.

### BACKGROUND ART

CD5 is a macromolecular glycoprotein mainly distributed in T lymphocytes and in a small number of B lymphocytes. It not only participates in the innate immune response, but also regulates the specific immune response mediated by T and B lymphocytes, and affects the biological characteristics and functions of T and B lymphocytes through the T lymphocyte receptor (TCR) and B lymphocyte receptor (BCR) signaling pathways. CD5 molecules can not only assist T cells in regulating various immune balances in autoimmune diseases, but also generate autoantibodies in B cells to clear some autopathological antigens, so as to participate in the process of various autoimmune diseases.

CD5 is a 67kDa type I transmembrane glycoprotein that structurally belongs to the ancient and highly conserved scavenger receptor cysteine-rich (SRCR) superfamily. SRCR superfamily molecules are able to mediate isotype or heterotype interactions, thus mediate molecular pattern recognition related to causative pathogens. The cysteine-rich CD5 is mainly composed of an extracellular region of 347 amino acid residues and an intracellular region of 93 amino acid residues[1]. The extracellular region contains three SRCR domains (D1, D2, and D3) and one hydrophobic transmembrane region, which can serve as a receptor for regulating T cell proliferation, so that the CD5 molecules are expected to be an ideal target of T cell malignancies. In particular, D3 is a juxtamembrane domain[2]. In studies of CAR-T cells targeting different antigens, it has been shown that binding to the juxtamembrane epitope of antigen molecules can enhance the immune function of CAR-T cells.

The single domain antibody (sdAb) is an artificially designed antibody molecule that is a naturally occurring heavy chain antibody lacking a light chain. The sdAb are found in camelid animals such as alpacas and dromedaries, as well as cartilaginous fish such as sharks and rays. The sdAb has a molecular weight that is only 1/10 that of ordinary antibodies. It is the smallest complete antigen-binding fragment. Therefore, sdAb is also known as nanobody. The long CDR3 region of the variable region of the sdAb heavy chain can form a stable convex ring structure. The stable disulfide bonds in this structure can penetrate the interior of the antigen. Compared with the concave topology formed by ordinary antibodies and antigens, VHH antibodies have a higher affinity for antigens. The sdAb has small molecular weights and long CDR3s, which makes sdAb more flexible and promotes the carrying of targeted drugs to binding sites that are difficult for traditional antibodies to reach in order to exert their effects. The sdAb has better solubility and are more hydrophilic than traditional antibodies. Since the sdAb contains more hydrophilic amino acids, it has greater thermal stability and still retains its biological activity under the action of proteases. According to the structure of the sdAb, it can be used to display hidden epitopes of conventional immunoglobulins, so that the sdAb has been widely used in fields such as immunological research, diagnostic detection, medical and bioimaging, and the development of therapeutic antibodies[3].

Currently, there are no marketed drugs targeting CD5-D3. Therefore, there is an urgent need to develop a therapeutic single domain antibody with high affinity, specific targeting of CD5 molecules, and ability to recognize the extracellular D3 juxtamembrane domain of CD5 molecules.
[1] He Xin, Xing Limin, Shao Zonghong. Research progress on CD5 and its role in autoimmune diseases [J]. Chinese Journal of Immunology, 2020, 36(14):1766-1770.
[2] Consuegra-Fernández M, Aranda F, Simões I, et al. CD5 as a Target for Immune-Based Therapies. Crit Rev Immunol[J]. 2015;35(2):85-115.
[3] Hamers-Casterman C, Atarhouch T, Muyldermans S, et al. Naturally occurring antibodies devoid of light chains[J]. Nature. 1993 Jun 3;363(6428):446-448.

### SUMMARY

In view of the above shortcomings, the present invention provides an anti-CD5 single domain antibody and application thereof. The provided anti-CD5 single domain antibody can specifically bind to CD5, and can recognize an extracellular region D3 juxtamembrane domain of a CD5 molecule. Further provided is a humanized anti-CD5 single domain antibody, which reduces the immunogenicity of a molecule in the body. The humanized CD5 single domain antibody can specifically recognize a CD5 molecule and has relatively high affinity. The present invention provides broad prospects for the development of drugs targeting CD5.

### Terms:

In this invention, the term "amino acid" includes natural amino acids, synthetic amino acids, and amino acid analogs and amino acid mimics that function in a manner similar to that of natural amino acids. Natural amino acids are amino acids encoded by the genetic code. Amino acid analogs are those that have the same basic chemical structure as naturally occurring amino acids. Amino acids may be mentioned herein by their commonly known three-letter symbols or by the single-letter symbols recommended by the IUPAC-IUB (International Union of Pure and Applied Chemistry - International Union of Biochemistry) Committee on Biochemical Nomenclature.

In this invention, the term "nucleotide sequence" refers to the arrangement of bases in DNA or RNA. That is, the arrangement of A, T, G, C in DNA, or the arrangement of A, U, G, C in mRNA, and also includes the arrangement of bases in rRNA, tRNA, and mRNA.

In this invention, the heavy chain refers to two longer identical heavy chains (H chains) with larger relative molecular weight in the antibody, the light chain refers to two shorter identical light chains (L chains) with smaller relative molecular weight molecular weight in the antibody.

In this invention, the variable region refers to the region of the immunoglobulin light chain and heavy chain near the N-terminus where the amino acid sequence changes significantly.

In the present invention, the term "complementary determining region" or "CDR" generally refers to the complementarity determining region within the variable region of an antigen binding fragment. In the present invention, the heavy chain variable region has three CDRs, which are named HCDR1, HCDR2 and HCDR3 for each variable region.

In the present invention, the term "FR" generally refers to the more highly conserved parts of antibody variable domains, which are called the framework regions.

In the present invention, the term "single domain antibody (sdAb)" or "VHH" generally refers to a type of antibody that lacks the antibody light chain and only has the heavy chain variable region.

In the present invention, the term "chimeric antigen receptor" or "CAR" generally refers to a group of polypeptides, usually two in the simplest embodiment, which provides the cell with specificity for a target cell (usually a cancer cell) and generate an intracellular signal when it was in immune effector cells. In some implementations, the CAR includes at least one extracellular antigen binding domain (such as a VHH, scFv or part thereof), a transmembrane domain and a cytoplasmic signaling domain (herein also referred to an "intracellular signaling domain") , which includes a functional signaling domain derived from a stimulatory molecule and/or a co-stimulatory molecule as defined below.

In this invention, the term "screening of human natural phage display antibody library" refers to the preliminary selection of human natural phage display antibody library using target proteins or overexpressed cell lines as antigens. Based on the preliminary selection results, a sufficient number of monoclone antibodies are selected for initial screening. Positive clones are selected for sequencing, and sequence diversity analysis is performed to select sequence-specific antibody clones. Antibody samples are then prepared. In this specification, "human natural phage display antibody library" and "human natural library" are used interchangeably.

In the present invention, the term "functional variant" generally refers to an amino acid sequence having substantially the same function as the wild type amino acid sequence and having at least 85% sequence identity with the wild type amino acid sequence.

In the present invention, the term "natural killer cell" or "NK cell" generally refers to a type of cytotoxic lymphocyte of the immune system.

In the present invention, the term "expression" generally refers to the transcription and/or translation of a specific nucleotide sequence.

In this invention, the term "vector" refers to a nucleic acid molecule capable of proliferating another nucleic acid linked to it. The term includes vectors that function as self-replicating nucleic acid structures and vectors that are incorporated into the genome of the host cell that receives them. Some vectors can guide the expression of nucleic acids that are operatively linked to them. This type of carrier is referred to as an "expression carrier" herein.

The term "humanized" antibody refers to the part of the constant region of the antibody (i.e., the CH and CL regions) or all the antibodies are encoded by human antibody genes. Humanized antibodies can greatly reduce the immune side effects caused by heterologous antibodies to the human body. Humanized antibodies include such as chimeric antibodies, modified antibodies, and fully humanized antibodies. It should be understood that those skilled in the art can prepare suitable humanized forms of the single domain antibodies of the present invention according to actual needs, which is within the scope of the present invention.

The technical proposal of the present invention includes:
In one aspect, the present invention provides an anti-CD5 single domain antibody comprising a heavy chain variable region. The heavy chain variable region comprising complementary determining regions CDR1, CDR2 and CDR3;
the CDR1 contains the amino acid sequence shown in SEQ ID NO: 1;
SEQ ID NO: 1: SYAMG.
the CDR1 contains the amino acid sequence shown in SEQ ID NO: 2;
SEQ ID NO: 2: GAIHKSGGDTYYADSVK.
the CDR1 contains the amino acid sequence shown in SEQ ID NO:3;
SEQ ID NO: 3: CAAGDGTDTWDEYDY.

Specifically, the amino acid sequence of the anti-CD5 single domain antibody comprises the amino acid sequence shown in SEQ ID NO: 4 with at least 80% homology.

SEQ ID NO: 4:
DVQLQASGGGLVQAGGSLRLSCAASGRTFSSYAMGWFRQAPGKEREFVGAIH
KSGGDTYYADSVKGRFTISRDNAKNTVYLQMNSLKPEDTAVYYCAAGDGTDTWDEYD
YWGQGTQVTVSS.

In another aspect, the present invention provides a nucleotide which encodes the above-mentioned anti-CD5 single domain antibody.

Specifically, the nucleotide sequence is shown in SEQ ID NO: 5; or at least 80% homology with the sequence shown in SEQ ID NO: 5.

SEQ ID NO: 5:
gatgtgcagctgcaggcgtctgggggaggattggtgcaggctgggggctctctgagactctcctgtgcagcctctggacgc
accttcagtagctatgccatgggctggttccgccaggctccagggaaggagcgtgagtttgtcggagctattcacaaaagtggtggtgaca
catactatgcagactccgtgaagggccgattcaccatctccagagacaacgccaagaacacggtgtatctgcaaatgaacagcctgaaac
ctgaggacacggccgtttattactgtgcagcaggggacgggaccgacacttgggatgagtatgactactggggccaggggacccaggtc
accgtctcctca.

In another aspect, the present invention provided an expression vector comprising the above-mentioned nucleotides.

Specifically, the expression vector is a prokaryotic cell expression vector or a eukaryotic cell expression vector.

Preferably, the expression vector is the pComb3xss vector.

In another aspect, the present invention provided a host cell comprising the above-mentioned expression vector.

Specifically, the host cell is a prokaryotic expression cell or a eukaryotic expression cell.

Preferably, the host cell is Escherichia coli.

In another aspect, the present invention provided a humanized anti-CD5 single domain antibody obtained by humanizing the anti-CD5 single domain antibody according to any one of claims 1-2.

Specifically, the amino acid sequence of the humanized anti-CD5 single domain antibody is shown in SEQ ID NO: 6; or at least 80% homology with the sequence shown in SEQ ID NO: 6.

SEQ ID NO: 6:
DVQLQASGGGLVQPGGSLRLSCAASGRTFSSYAMGWFRQAPGKGLEFVGAIH
KSGGDTYYADSVKGRFTISRDNSKNTVYLQMNSLRAEDTAVYYCAAGDGTDTWDEYD
YWGQGTLVTVSS.

In another aspect, the present invention provides a nucleotide which encodes a humanized anti-CD5 single domain antibody.

Specifically, the nucleotide sequence is shown in SEQ ID NO: 7; or at least 80% homology with the sequence shown in SEQ ID NO: 7.

SEQ ID NO: 7:

gatgttcaactgcaagctagtggtggaggtctggtccaacccggagggtccctccggctgagctgtgctgccagcggacgc

acattctcttcttatgctatgggttggttccggcaggcacccggcaaagggctggagttcgtgggagctatccataagtccggaggagacac

ctactacgctgacagtgtcaagggccggttcaccataagcagggataacagcaagaacaccgtctacctccagatgaacagcctcagggc

tgaagacacagccgtgtactattgtgccgcaggcgacggcactgacacatgggacgagtacgattattggggtcagggcaccctggtcac

tgtgtcctcc.

In another aspect, the present invention provides an expression vector comprising the above-mentioned nucleotide sequence.

Specifically, the expression vector is a prokaryotic cell expression vector or a eukaryotic cell expression vector.

Preferably, the expression vector is the IG17479-1 01H-PTT5-hFc vector.

In another aspect, the present invention provides a host cell comprising the above-mentioned expression vector.

Specifically, the host cell is a prokaryotic expression cell or a eukaryotic expression cell.

Preferably, the host cell is a 293TS cell.

In another aspect, the present invention provides a use of the aforementioned anti-CD5 single domain antibody in drug preparation.

Specifically, the use comprises the aforementioned anti-CD5 single domain antibody; nucleotide; expression vector and/or host cell.

Specifically, the use comprises those used in the preparation of drugs to combat cancer or autoimmune diseases.

More specifically, the cancer comprises T-cell malignancies or B-cell malignancies.

Preferably, the T-cell malignancies comprise acute lymphoblastic leukemia, T-cell large granular lymphoblastic leukemia, adult T-cell leukemia/lymphoma, T-cell pre-lymphoblastic leukemia, or peripheral T-cell lymphoma.

Preferably, the B-cell malignancies comprise non-Hodgkin's lymphoma or chronic lymphocytic leukemia.

More specifically, the autoimmune diseases comprise rheumatoid arthritis or graft-versus-host disease.

The beneficial effects of the invention are in that:
The provided anti-CD5 single domain antibody can specifically bind to CD5 and has relatively high affinity.

The provided anti-CD5 single domain antibody can recognize an extracellular region D3 juxtamembrane domain of a CD5 molecule, which can enhance the immune function of CAR-T cells.

The further provided humanized anti-CD5 single domain antibody reduces the immunogenicity of a molecule in the body. The humanized anti-CD5 single domain antibody can specifically recognize a CD5 molecule and has relatively high affinity.

### BRIEF DESCRIPTION OF THE DRAWINGS

FIG. 1 is the electrophoresis diagram of single domain antibody genes in the first round of PCR.
FIG. 2 is the electrophoresis diagram of single domain antibody genes in the second round of PCR.
FIG. 3 is the binding reaction of CD5 single domain antibody to CD5-positive cells Jurkat detected by FACS (flow cytometry).
FIG. 4 is the binding reaction of 12C single domain antibody with CD5 positive cells Jurkat, and CD5 negative cells Raji detected by FACS.
FIG. 5 is the binding reaction of 12C single domain antibody to 293TS-CD5D3 cells detected by FACS.
FIG. 6 is the binding and dissociation curves and fitting diagrams of the 12C single domain antibody and the CD5 truncated protein.
FIG. 7 is the affinity level of the 12C single domain antibody binding to target cells.
FIG. 8 is the binding reaction of humanized 12C-huVHH antibody with CD5 positive cells Jurkat, CD5 overexpressing cells 293T-CD5, and CD5 negative cells Raji detected by FACS.
FIG. 9 is the affinity level of the humanized 12C-huVHH antibody binding to target cells.

### DETAILED DESCRIPTION

The invention will be described below with specific examples, which are not used to limit the invention, but only to illustrate the invention, so as to make the technical scheme of the invention easier to understand and master. Unless otherwise specified, the experimental methods described in the following embodiments are conventional methods; the reagents and materials described are commercially available unless otherwise specified.

The experimental materials used in this invention are shown in Table 1:

**Table 1 Experimental Materials**

| Experimental instruments and materials | Manufacturer | Item number |
|---|---|---|
| Human CD5(Arg25-Pro372)-His protein | Sinobiological | 11027-H08H |
| QIAGEN Kits | QIAGEN | 52304 |
| PrimeScript^{™} II 1st Strand cDNA Synthesis Kit | TaKaRa | 6210A |
| *BamHI* endonuclease | NEB | R3136L |
| *XhoI* endonuclease | NEB | R0146L |
| *BBSI* endonuclease | NEB | R3539L |
| pComb3xss vector | BioVector NTCC Inc. | BioVector-pComb3XSS |
| helper phage M13KO7 | NEB | N0315S |
| 293TS cells | ATCC | CRL-3216 |

### Example 1 Construction of an anti-CD5 antigen-specific antibody library

### 1. Immunizing alpacas with CD5 antigen

The human CD5 (Arg25-Pro372)-His is used for the immune and serum titer tests for alpacas. A healthy adult alpaca was selected and vaccinated by injecting antigens subcutaneously at multiple points on the back of the neck. The vaccination was repeated six times and the absorption of the lumps at the injection sites was monitored to confirm that the immunization was correct. The immunization interval was two weeks. After the fifth immunization, serum was collected to determine the immune titer of antigen. When the titer reached more than 10,000 times (ELISA (Enzyme-Linked Immunosorbent Assay) method), about 100 mL of whole blood was collected, lymphocytes were separated, and stored at -80°C for later use.

### 2. Construction of a phage antibody library and screening for CD5 single domain antibodies specific to the antigen

(1) the QIAGEN kit was used to extract RNA from lysed lymphocytes according to the instructions, and the RNA was purified;
(2) the RNA was reversed to cDNA using the PrimeScript^{™} II 1st Strand cDNA Synthesis Kit to construct a cDNA library;
(3) the target gene was cloned using nested PCR;

Nested PCR was used to amplify the antibody heavy chain variable region (VHH) gene fragment in two rounds using two sets of primers.

### 1) the first round of PCR amplification was performed:

PCR primers were constructed:

**Table 2. Downstream primers for the first round of PCR amplification**

| Primers | Serial number | Sequence |
|---|---|---|
| YT-1 | SEQ ID NO: 8: | CGCCATCAAGGTACCAGTTGA |
| YT-2 | SEQ ID NO: 9: | GGGGTACCTGTCATCCACGGACCAGCTGA |
| YT-3 | SEQ ID NO: 10: | TACTTCATTCGTTCCTGAGGAGACGGT |
| YT-4 | SEQ ID NO: 11: | GGAGCTGGGGTCTTCGCTGTGGTGCG |
| YT-5 | SEQ ID NO: 12: | TGGTTGTGGTTTTGGTGTCTTGGGTT |

**Table 3. Upstream primers for the first round of PCR amplification**

| Primers | Serial number | Sequence |
|---|---|---|
| YT1BN-BamHI | SEQ ID NO: 13: | CGGGATCCSMKGTRCAGCTGGTGGAKT CTGGGGGAG |
| YT2BN-BamHI | SEQ ID NO: 14: | CGGGATCCCAGGTAAAGCTGGAGGAGT CTGGGGGAG |

**Table 4 Primer set for the first round of PCR amplification**

| Primers set | F | R |
|---|---|---|
| 1# | YT1BN-BamHI, YT2BN-BamHI | YT-1, YT-2 |
| 2# | YT1BN-BamHI, YT2BN-BamHI | YT-3, YT-4 |
| 3# | YT1BN-BamHI, YT2BN-BamHI | YT-5 |

In the table, F and R are amplification primers, where F represents the forward primer and R represents the reverse primer.

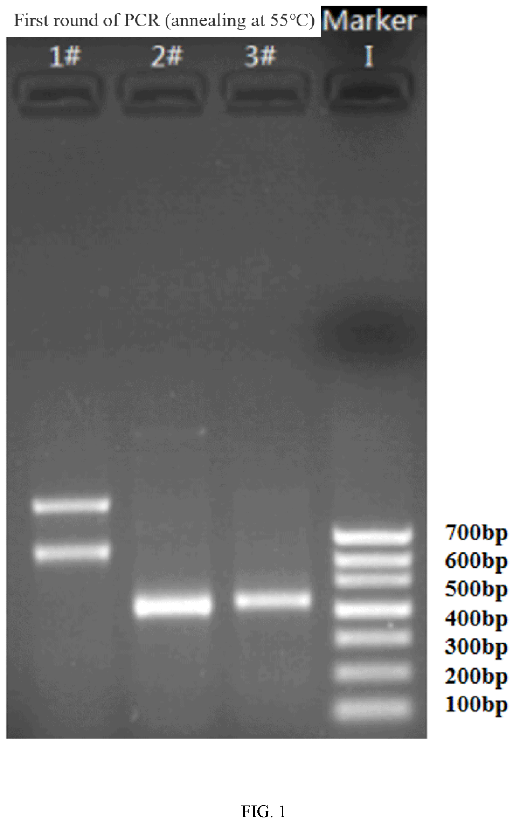

The first round of PCR amplification yielded ordinary heavy chain antibody gene fragments larger than 700 bp, heavy chain antibody gene fragments missing the light chain between 500 bp and 700 bp, and antibody heavy chain variable region (VHH) target gene fragments between 400 bp and 500 bp. Gene fragments of 500bp-700bp and 400bp-500bp were screened out and recovered by gel electrophoresis analysis. The electrophoresis results are shown in FIG. 1. The PCR products of primer group 1# show two bright bands. One band is larger than 700bp, which represents ordinary antibody DNA, and the other band is between 500bp and 700bp, which represents heavy chain antibody DNA. The PCR products of primer group 2 and primer group 3 were variable region fragments (VHH) of heavy chain antibodies, with a size between 400bp and 500bp. The heavy chain antibody gene fragment missing the light chain between 500bp and 700bp and the target gene fragment of the antibody heavy chain variable region (VHH) between 400bp and 500bp were gel-excised and recovered.

### 2) the second round of amplification was performed:

Primers were constructed:

**Table 5 Downstream primers for the second round of PCR amplification**

| Primers | Serial number | Sequence |
|---|---|---|
| YTV8-XhoI | SEQ ID NO: 15: | CCGCTCGAGTGAGGAGACGGTGACCTGG |

**Table 6 Upstream primers for the second round of PCR amplification**

| Primers | Serial number | Sequence |
|---|---|---|
| SDABL-1-BamHI | SEQ ID NO: 16: | CGGGATCCSMKGTRCAGCTGGTGGAKTC TGGGGGAG |
| SDABL-2-BamHI | SEQ ID NO: 17: | CGGGATCCCAGGTAAAGCTGGAGGAGT CTGGGGGAG |
| SDABL-3-BamHI | SEQ ID NO: 18: | CGGGATCCGAKGTGCAGCTGCAGGCGT CTGGGGGAG |

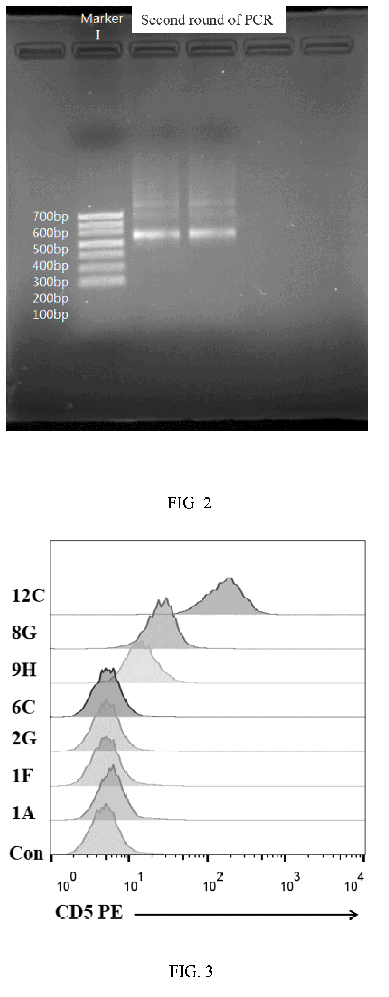

The target gene in the variable region (VHH) of the antibody heavy chain was amplificated. The recovered complete heavy chain antibody and its heavy chain variable region gene fragment were used as templates, the target gene (400bp-500bp) of antibody heavy chain variable region (VHH) was obtained by the second round of PCR amplification with antibody heavy chain variable region (VHH) specific primers. The electrophoresis results are shown in FIG. 2. A bright band can be seen, and the target gene of the antibody heavy chain variable region (VHH) is between 400bp and 500bp. That is, the bright band contains multiple target genes of the antibody heavy chain variable region (VHH) between 400bp and 500bp. In the second round of PCR amplification, gel electrophoresis was used to screen for target genes containing the antibody heavy chain variable region (VHH) (i.e., gene fragments between 400bp and 500bp).

### (4) Construction of the target gene library

The antibody heavy chain variable region (VHH) target gene fragment obtained above and the phage vector pComb3xss were enzyme-digested with *BamHI* and *XhoI* endonuclease. After digestion, the antibody heavy chain variable region (VHH) target gene fragment was ligated into the pComb3xss vector using a ligase to construct a recombinant plasmid. The constructed recombinant plasmid was electroporated into TG1 competent cells to enable the target gene fragment of the antibody heavy chain variable region (VHH) to be fused and expressed with the pComb3xss vector. The storage capacity was calculated to be 1.58 × 10⁸ using gradient dilution plating. To detect the insertion rate of the library, 48 clones were randomly selected for colony PCR. The results showed that the insertion rate reached 94%.

### (5) Expand cultivation

The bacterial seed containing bacteriophage stored at -80°C was thawed at room temperature, mixed well, and 500 µL was taken and added to 70 mL of 2YT medium. Helper bacteriophage M13KO7 was added for infection, and the culture was carried out overnight. After centrifugation, the supernatant was collected and 20% PEG-2.5M NaCl was added and mixed well (the bacteriophage was in the supernatant). The precipitate was collected by centrifugation, and PBS and glycerol were added for resuspending, and stored at -80°C for later use.

### Example 2 Screening of specific phages

Since there were multiple types of heavy chain antibody variable region (VHH) fragments amplified by nested PCR, and not all of these gene fragments were Anti-CD5 target fragments, after these VHH fragments were transformed into phages, the target phages needed to be purified. The steps for purifying the target phages were as follows:
1. Diluting CD5 protein to 100 µg/mL with coating buffer and coating with 150 µL/well. After standing at room temperature for 2 hours, incubating overnight at 4°C.
2. Aspirating the coating solution, adding 300µL/well of blocking solution, and incubating at 37°C for 2 hours;
3. Aspirating the blocking solution, adding the phage antibody library (5×10¹¹ - 1×10¹²), and standing at room temperature for 2 hours;
4. Washing the sieve wells 10 times respectively with PBST (containing 0.05% Tween 20) and PBS for 2 minutes each time to remove unbound phages;
5. Adding TEA into the sieve wells to wash out the phage, resuspending evenly by blowing and aspiration, and standing at room temperature for 10 minutes;
6. After resuspending evenly by blowing and aspiration again, adding to pre-cooled 1M Tris-HCl and mixing well, then determining the titer.
7. Amplifying and purifying the amplified phage.

The above steps 1-7 were repeated four times, and the phage prepared in step 7 was used as the phage added to the micropores in step 3 of the next round (the first three rounds were full-length CD5 protein (Arg25-Pro372)-His, and the fourth round was CD5 truncated protein (Ser 276-Gln368)-FC, the first screening coating concentration was 100µg/mL, the second screening coating concentration was 10µg/mL, the third screening coating concentration was 1µg/mL, and the fourth screening coating concentration was 10µg/mL). As the number of selection rounds increased, the coating concentration decreased stepwise, but the number of eluted phages increased, thus highly enriched CD5-specific phage was obtained.

### Example 3: Screening for specific positive monoclonal antibodies

1. Performing PCR amplification on the above CDS-specific phages that have been enriched to obtain a specific CD5 single domain antibody gene (a PCR product with restriction endonuclease *BbsI* and *BamHI* sites);

The primers used for PCR amplification are shown in Tables 7 and 8:

**Table 7 Downstream primers for PCR amplification**

| Primers | Serial number | Sequence |
|---|---|---|
| R | SEQ ID NO: 19: | gAAgATCTCCggATCCTgAggAgACggTgACCTgggT |

In the table, F and R are amplification primers, where F represents the forward primer and R represents the reverse primer.

**Table 8 Upstream primers for PCR amplification**

| Primers | Serial number | Sequence |
|---|---|---|
| Sd (1) | SEQ ID NO: 20: | AAGAAGAAGACAACAGGCCSAGGTACAGCTGGTGG AATCTGGGG |
| Sd (2) | SEQ ID NO: 21: | GAAGAAGAAGACAACAGGCCCAGGTAAAGCTGGAGG AGTCTGGG |
| Sd (3) | SEQ ID NO: 22: | GAAGAAGAAGACAACAGGCCGAKGTGCAGCTGCAGG GCGTCTGGG |

2. Treating the PCR product prepared in step 1 and pSJF2 vector with restriction endonucleases *BbsI* and *BamHI,* respectively, and then ligating and recombining with T4 ligase to obtain the plasmid sdAb-pSJF2, which can be efficiently expressed in Escherichia coli;

3. Randomly picking a plurality of single colonies from the agar plate on which the colonies were growing, and then inoculating them into a 96-well deep-well culture plate containing 2YT liquid medium containing Amp.

4. After culturing for 4 hours, inoculating the individual clones one by one onto numbered LB solid plates containing Amp separated by small grids;

5. Adding IPTG to the deep-well culture plate to a final concentration of 0.5 mM for induction;

6. After culturing overnight, harvesting the bacterial supernatant expressing the protein;

7. Performing ELISA assay using CD5 antigen and selecting the ELISA results of Anti-CD5 positive clones.

**Table 9 ELISA Detection Results**

| | 1 | 2 | 3 | 4 | 5 | 6 | 7 | 8 | 9 | 10 | 11 | 12 |
|---|---|---|---|---|---|---|---|---|---|---|---|---|
| A | 4.039 | 3.870 | 3.787 | 3.788 | 4.002 | 3.685 | 3.479 | 3.697 | 0.070 | 3.740 | 3.734 | 3.245 |
| B | 4.000 | 3.714 | 4.024 | 3.801 | 3.843 | 3.897 | 3.894 | 4.246 | 3.760 | 3.847 | 3.913 | 3.940 |
| C | 3.944 | 3.945 | 3.958 | 3.977 | 3.982 | 3.823 | 4.010 | 3.847 | 3.898 | 3.983 | 4.172 | 3.936 |
| D | 3.957 | 3.803 | 3.935 | 3.807 | 3.872 | 3.664 | 3.917 | 4.160 | 3.731 | 3.906 | 3.990 | 3.821 |
| E | 3.863 | 3.934 | 3.890 | 3.774 | 3.980 | 3.158 | 3.797 | 3.663 | 3.839 | 3.857 | 4.039 | 3.478 |
| F | 3.936 | 3.979 | 3.960 | 3.916 | 3.749 | 3.955 | 3.699 | 3.803 | 3.828 | 3.912 | 3.696 | 3.805 |
| G | 3.987 | 3.909 | 4.079 | 3.978 | 3.858 | 3.887 | 3.837 | 3.866 | 3.836 | 3.906 | 3.839 | 0.064 |
| H | 3.910 | 4.112 | 3.797 | 3.827 | 3.778 | 3.730 | 0.082 | 3.640 | 3.977 | 3.575 | 3.676 | 0.048 |

The ELISA results are shown in Table 9: consistent with the 96-well deep plate, the first column A-H is the vertical column number, and the first row 1-12 is the horizontal column number. The clones were numbered and located by using the horizontal and vertical numbers as coordinates (A1-H12), and the clones in the deep plate were matched with the OD₄₅₀ₙₘ detection readings. 94 clones were inoculated in each plate (G12 and H12 were left uninoculated as negative controls). Except for 9A and 7H, which were negative clones, the rest were positive clones. Positive clones were sequenced, and the sequencing results were analyzed to obtain the gene sequence of the variable region (VHH) of the heavy chain of positive candidate antibodies.

### Example 4: Expression and purification of CD5 single domain antibody in host Escherichia coli

After obtaining the above positive monoclonal antibody, it needed to be expressed to obtain CD5 single domain antibody. The subsequent expression was mainly carried out in Escherichia coli, then purified to obtain the desired CD5 single domain antibody. The specific operation process is as follows:
(1) Based on the diversity analysis of ELISA positive sequences, the seven representative positive clone media (1A, 1F, 2G, 6C, 8G, 9H, 12C) selected in Example 3 were inoculated into 4 mL of LB culture medium containing ampicillin and cultured overnight at 37°C in a shaker.
(2) 1 mL of overnight culture was transferred to 100 mL of LB medium containing ampicillin and cultured at 37°C and 230 rpm on a shaker. When the OD value reached 0.4-0.6, 1.0 mM IPTG was added and continued to culture overnight, then centrifuge and collect the bacteria. The bacteria were lysed using the hyperosmolar method, centrifuged, and collected the soluble single domain antibody protein in the supernatant.
(4) Proteins with a purity of over 95% were obtained by Ni+ ion affinity chromatography.

### Example 5 Flow cytometry assay to detect the binding of CD5 single domain antibody to human CD5-expressing cells

In order to evaluate the binding activity of CD5 candidate antibodies on Jurkat cells of a T-cell acute lymphoblastic leukemia cell line expressing CD5 molecules, the binding activity to CD5 protein on the cells was detected using the FACS method. The results are shown in FIG. 3.

As shown in FIG. 3, clones 1A, 1F, 2G, 6C, 8G, and 9H do not bind to positive Jurkat cells or the binding is weak. The single domain antibody candidate molecule with clone number 12C has the strongest binding activity on Jurkat cells of the T-cell acute lymphoblastic leukemia cell line expressing CD5 molecules.

### Example 6 Flow cytometry assay to detect the specificity of 12C single domain antibody

In order to further analyze the specificity of the initial screening clone 12C single domain antibody and determine the best candidate clone, FACS was used to detect the binding reactivity of 12C antibody with CD5 positive cells Jurkat and CD5 negative cells Raji.

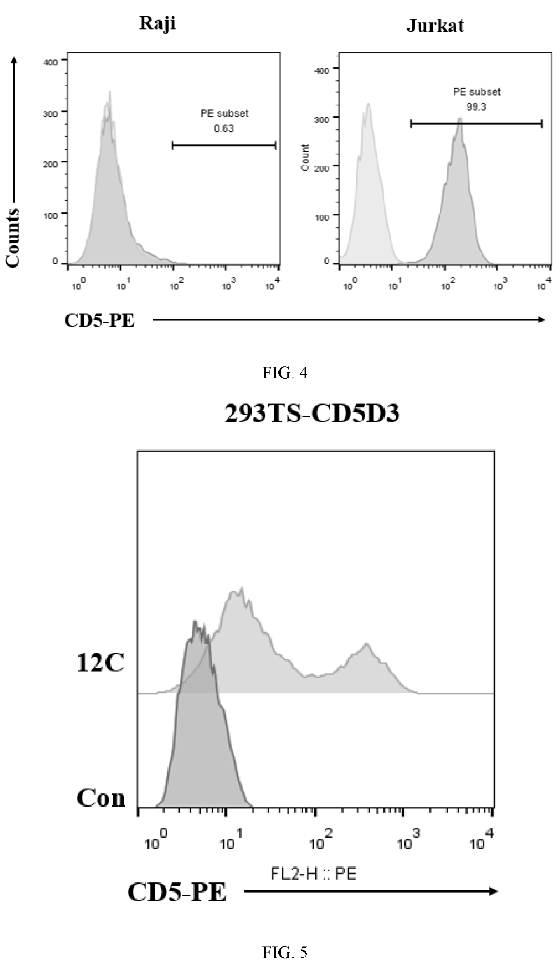

As shown in FIG. 4, the 12C single domain antibody can bind to CD5 positive Jurkat cells, but not to CD5 negative Raji cells, which indicates that the 12C single domain antibody is a specific clone.

### Example 7 12C single domain antibody recognizes the extracellular D3 juxtamembrane domain (CD5-D3) of the CD5 molecule

The CD5 molecule has three extracellular domains (D1, D2, and D3), of which D3 is the juxtamembrane domain with the amino acid sequence Ser276-Gln368. The human CD5 truncated protein (CD5-D3) gene sequence was synthesized through gene synthesis, constructed into a vector, ligated, and introduced into Escherichia coli. Single clones of Escherichia coli were picked and sequenced to obtain the correct plasmid clones, which were then extracted. 293TS cells in the logarithmic growth phase were transiently transfected using the transfection reagent PEI. The binding activity of 12C single domain antibody to 293TS-CD5D3 cells was detected by FACS. As shown in FIG. 5, the 12C single domain antibody was able to bind to 293TS-CD5D3 cells, which indicated that the 12C single domain antibody can recognize the extracellular D3 juxtamembrane domain of the CD5 molecule.

### Example 8 Protein level affinity determination experiment

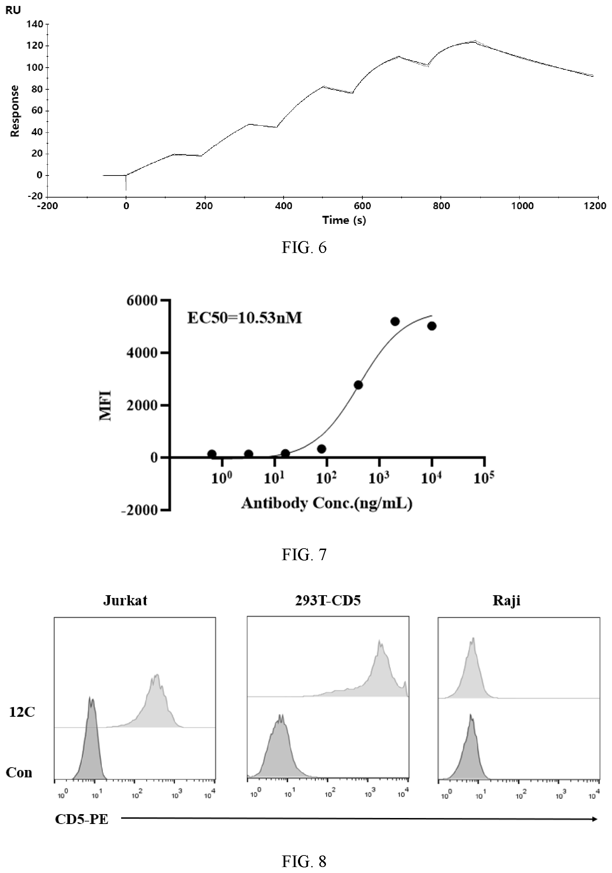

The binding affinity of CD5 -VHH to human CD5 truncated protein was detected by surface plasmon resonance (SPR). Human CD5 truncated protein was coupled to the CM5 chip to capture CD5 -VHH antibody. Affinity was detected using 12C single domain antibody as the mobile phase, and the results are shown in FIG. 6.

FIG. 6 is the binding and dissociation curves and fitting diagrams of the 12C single domain antibody and the CD5 truncated protein. The affinity value of 12C single domain antibody and antigen was obtained by calculation and analysis. Affinity data are shown in Table 10. The results showed that the 12C single domain antibody was a high-affinity anti-human CD5 antibody with an affinity KD value reaching the nm level.

**Table 10 Antibody SPR Detection Results**

| Antibodies | Receptor | ka (1/Ms) | kd (1/s) | KD (M) |
|---|---|---|---|---|
| 12C | Human CD5 truncated protein | 3.10E+05 | 9.65E-04 | 3.12E-09 |

### Example 9 Expression, purification, and cellular affinity assay of CD5 single domain antibody in eukaryotic cells

### 1. Expression and purification of CD5 single domain antibodies in eukaryotic cells

The single domain antibody 12C-VHH sequence was inserted into the IG17479-1 01H-PTT5-hFc vector plasmid and expressed using the 293TS cell expression system. One week after expression, the supernatant was collected, and purified by Protein A GE, then quantitatively analyzed using Nanodrop assay, and performed by SDS-PAGE analysis.

### 2. Cell level affinity detection

The 12C-VHH antibody was serially diluted with FACS dilution buffer and co-incubated with Jurkat cells. The antibody was detected as anti-human IgG Fc PE. The EC₅₀ of the antibody was calculated by fitting the curve, and the results are shown in FIG. 7. FIG. 7 shows that the EC₅₀ value of the 12C single domain antibody binding to CD5 on the surface of Jurkat cells is 10.53 nM.

### Example 10 Candidate VHH Sequence

After characterization through the above steps, a single domain antibody 12C exhibiting high affinity, good specificity, and recognition of the D3 juxtamembrane domain of the CD5 molecule's extracellular region was obtained. The amino acid sequence of the 12C single domain antibody is shown in SEQ ID NO: 4. Its complementarity-determining regions CDR1, CDR2, and CDR3 are shown in Table 11, and its nucleotide sequence is shown in SEQ ID NO: 5.

**Table 11 Information on the CDR region of VHH antibody**

| CDR region | SEQ ID | Sequence |
|---|---|---|
| CDR1 | SEQ ID NO: 1: | SYAMG |
| CDR2 | SEQ ID NO: 2: | VGAIHKSGGDTYYADSVK |
| CDR3 | SEQ ID NO: 3: | CAAGDGTDTWDEYDY |

### Example 11 Antibody Humanization

In order to reduce the immunogenicity of the molecules in vivo, the present invention humanized the candidate molecules. Humanized molecules were obtained, and the humanized molecules were numbered as 12C-huVHH, respectively. The amino acid sequence of 12C-huVHH is shown in SEQ ID NO: 6. After codon optimization for expression in human cells, the nucleotide sequence is shown in SEQ ID NO: 7.

### Example 12 Expression and purification of humanized CD5 single domain antibody in eukaryotic cells

The humanized single domain antibody 12C-huVHH sequence was inserted into the IG17479-1 01H-PTT5-hFc vector plasmid and expressed using the 293TS cell expression system. One week after expression, the supernatant was collected, and purified by Protein A GE, then quantitatively analyzed using Nanodrop assay, and performed by SDS-PAGE analysis.

### Example 13 Flow cytometry assay to detect the specificity of humanized 12C single domain antibody

In order to evaluate the impact of humanization on the specificity of 12C single domain antibodies, the derived molecules after humanization were detected by FACS. The binding reactivity of 12C-huVHH to CD5-positive cells Jurkat, CD5-overexpressing cells 293T-CD5, and CD5-negative cells Raji was detected by flow cytometry (FACS), and the results are shown in FIG. 8.

The results showed that the humanized 12C single domain antibody could bind to CD5 positive cells Jurkat and 293T-CD5, but not to CD5 negative cells Raji. It shows that humanized 12C can specifically recognize CD5 molecule.

### Example 14 Cell-level affinity detection of derived molecules after humanization modification

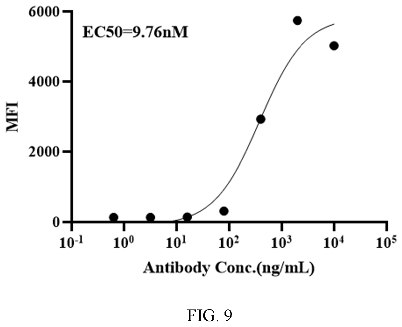

In order to evaluate the effect of humanization on antibody binding to human CD5-positive Jurkat cells, FACS was used to detect the derived molecules after humanization. The serially diluted antibody was co-incubated with CD5-expressing Jurkat cells, and the antibody was detected as anti-human IgG Fc PE. The EC₅₀ of the antibody was calculated by fitting the curve. The results are shown in FIG. 9. The results indicate that the humanized derivative molecule 12C-huVHH has a binding affinity of 9.76 nM to CD5 on the surface of Jurkat cells.

In summary, the technical solution of this invention provides a single domain antibody that specifically recognizes the D3 juxtamembrane domain of the extracellular region of the CD5 molecule by combining genetic engineering methods. This antibody exhibits high specificity and affinity.

### Comparative Example 1 compared with existing anti-CD5 single domain antibodies

**Table 12 Comparison with existing anti-CD5 single domain antibodies**

| Antibodies | Source | KD (M) | Jurkat binding ability | Features |
|---|---|---|---|---|
| 12C single domain antibody | The present invention | 3.12E-09 | Binding rate 99.3% | Specific binding to the D3 juxtamembrane domain |
| #62 IgG | CN116829600A | 1.67E-09 | Binding | NA |
| S005-NB144-73 | WO2022127844A1 | 2.48 E-10 | NA | NA |

| | | | | |
|---|---|---|---|---|
| Note: NA in the table represents not recorded. | | | | |

The above results show that, the provided anti-CD5 single domain antibody can specifically bind to CD5, has strong binding ability to Jurkat, and can recognize an extracellular region D3 juxtamembrane domain of a CD5 molecule.

The above detailed description is a specific description of one feasible embodiment of the present invention. The embodiment is not intended to limit the patent scope of the present invention. It should be pointed out that, any equivalent implementation or modification that does not deviate from the present invention should be included in the scope of the technical solution of the present invention. Therefore, the scope of protection of this invention shall be determined by the appended claims.

## Claims

1. An anti-CD5 single domain antibody, **characterized in that**, the anti-CD5 single domain antibody comprises a heavy chain variable region, and the heavy chain variable region comprises complementary determining regions CDR1, CDR2 and CDR3, the CDR1 comprises an amino acid sequence shown in SEQ ID NO: 1, the CDR2 comprises an amino acid sequence shown in SEQ ID NO: 2, the CDR3 comprises an amino acid sequence shown in SEQ ID NO: 3.

2. The anti-CD5 single domain antibody according to claim 1, **characterized in that**, an amino acid sequence of the anti-CD5 single domain antibody comprises an amino acid sequence shown in SEQ ID NO: 4.

3. A nucleotide, **characterized in that**, the nucleotide encodes the anti-CD5 single domain antibody according to any one of claims 1-2.

4. The nucleotide according to claim 3, **characterized in that**, a sequence of the nucleotide is shown in SEQ ID NO: 5.

5. An expression vector, **characterized in that**, the expression vector comprises the nucleotides according to any one of claims 3-4.

6. A host cell, **characterized in that**, the host cell comprises the expression vector of claim 5.

7. The host cell according to claim 6, **characterized in that**, the host cell is a prokaryotic expression cell or a eukaryotic expression cell.

8. A humanized anti-CD5 single domain antibody, **characterized in that**, the humanized anti-CD5 single domain antibody is obtained by humanizing the anti-CD5 single domain antibody according to any one of claims 1-2.

9. The humanized anti-CD5 single domain antibody according to claim 8, **characterized in that**, an amino acid sequence of the humanized anti-CD5 single domain antibody is shown in SEQ ID NO: 6.

10. A nucleotide encoding a humanized anti-CD5 single domain antibody.

11. The nucleotide according to claim 10, **characterized in that**, a nucleotide sequence of the nucleotide is shown in SEQ ID NO: 7.

12. An expression vector comprising the nucleotides according to any one of claims 10-11.

13. A host cell comprising the expression vector of claim 12.

14. A use of an anti-CD5 single domain antibody in drug preparation.

15. The use according to claim 14, **characterized in that**, the drug comprises the anti-CD5 single domain antibody according to any one of claims 1-2, or the nucleotide according to any one of claims 3-4, or the expression vector according to claim 5, or the host cell according to any one of claims 6-7, or the humanized anti-CD5 single domain antibody according to any one of claims 8-9, or the nucleotide according to any one of claims 10-11, or the expression vector according to claim 12, or the host cell according to claim 13.

16. The use according to claim 14, **characterized in that**, the drug comprises a drug for treating cancer or an autoimmune disease.
